# EUROPEAN PATENT APPLICATION

(11) **EP 1 674 868 A1**
(43) Date of publication of application: **28.06.2006**
(21) Application number: 04030375.2
(22) Date of filing: 21.12.2004
(51) Int. Cl.: G01N 33/569

(54) **Method and device for detection of mycobacterium tuberculosis antigens in biological fluids**

(71) Applicant: Chang Gung University (a university of Taiwan), Kwei-Shan Tao-Yuan (TW)
(72) Inventor: Chan, Err-Cheng, Kwei-Shan Tao-Yuan (TW); Yang, Ming-Ying, Kwei-Shan Tao-Yuan (TW)
(74) Representative: Hellmich, Wolfgang

(57) **Abstract**

A method and device for detection of mycobacterium tuberculosis antigens in biological fluids provides immunoassay methods, diagnostic kits, and an immunochromatoraphic assay device for detection of Mycobacterium tuberculosis antigens in biological specimens, preferably body fluids and tissues. The preferred body fluids are blood, serum, plasma, urine, pulmonary fluid, sputum, cerebrospinal fluid, and the preferred tissue is the lung biopsy specimen. The immunoassays require two primary antibodies against RD1, RD2, or RD3 of Mycobacterium tuberculosis. At least one of the primary antibodies is attached to a solid carrier. Optional, a second antibody against an animal species producing one of the primary antibodies can be added. Either the other primary antibody or the secondary antibody is labeled with a detection agent, which can be an enzymatic marker, a fluorescent or luminescent agent, a radio active label or a color particle. The biological specimens may be used directly, concentrated or diluted for the immunoassays.

## Description

The present invention relates to a method and device for detection of mycobacterium tuberculosis antigens, and more particularly to a method and device for detection of mycobacterium tuberculosis antigens in biological fluids.

Tuberculosis (TB) is caused by repeated exposure to airborne droplets contaminated with a rod-shape bacterium, *Mycobacterium tuberculosis.* The TB bacterium is also known as the tubercle bacillus. A person with active pulmonary tuberculosis can spread the disease by coughing and sneezing. Once the person is infected with *Mycobacterium tuberculosis,* the infection will slowly progress to disease. More than 8 million new cases of Tuberculosis (TB) have been diagnosed each year and are responsible for more than three million deaths per year. Almost two and three quarter billion people (2.75 billion) or 33% of population are latently infected with TB. Give the large incidence of TB and the TB associated disease, it is not surprising than there are many companies offering products for the diagnosis TB infection.

Among screening infectious diseases, a rapid and accurate diagnostic method of tuberculosis is very important for human health maintain and the disease control. At present, the clinical check (X-ray) coupling with the microscope examination and specimen bacterial culture is the major diagnostic method for the tuberculosis. However, this often takes 8 weeks, and the results sometime inaccurate. In the early days, the antibody detection in the blood test was regarded as a convenient method. However, its sensitivity and specificity appeared very poor. For the last 50 years, the successful program for screening tuberculosis is based on the tuberculin skin test (TST) by using the purified protein derivates (PPD) to stimulate the T cells. However, the major drawback of TST with PPD is its cross-reaction with BCG immunized persons.

The present invention has arisen to mitigate and/or obviate the disadvantages of the conventional method for detection of mycobacterium tuberculosis antigens.

The main objective of the present invention is to provide an improved method and device for detection of mycobacterium tuberculosis antigens in biological fluids.

This object is achieved by the subject matter of the independent claims.

Preferred embodiments are the subject matter of the dependent claims.

To achieve the objective, the method in accordance with the present invention may comprise the following steps.
1. Antigens and their antibodies preparations are derived from RD1, RD2, or RD3 gene cluster (region of difference) of *M*. *tuberculosis.*
2. Antigens can be produced both by purifying from *M*. *tuberculosis* cultures and genetic engineering strains of recombinant *E. coli*.
3. Recombinant proteins are combinations of single proteins and fused protein of RD1, RD2, or RD3 natures.
4. The preferred antigens are CFP-10 and ESAT-6, and CFP10/ESAT-6 fusion proteins encoded from the RD1 of *M. tuberculosis.*
5. Antibodies both monoclonal and polyclonal forms are derived from RDs proteins, RDs proteins fragments, and synthetic peptides encoded from any antigenic region of RDs proteins.
6. The preferred antibodies are derived from CFP-10 and ESAT-6, and CFP10/ESAT-6 fusion proteins.
7. Immunoassay devices include immuno-chromatographic assay device, enzyme immunoassay, latex-agglutination, fluorescence immunoassays, immuno-blotting and dot immuno-binging, luminescence immunoassay, immuno-agglutination, antibody chips.
8. The preferred embodiment of the immunoassay is the immunochromatographic assay device that first antibody bounded to a membrane and the second antibody is labeled with a color particle or a fluorescence material.
9. The immunoassay devices in this invention can detect the *Mycobacterium tuberculosis* antigens in blood, blood plasma, blood serum, sputum, urine, cerebrospinal fluid, pulmonary fluid, and tissues fluids.

Further benefits and advantages of the present invention will become apparent after a careful reading of the detailed description with appropriate reference to the accompanying drawings.

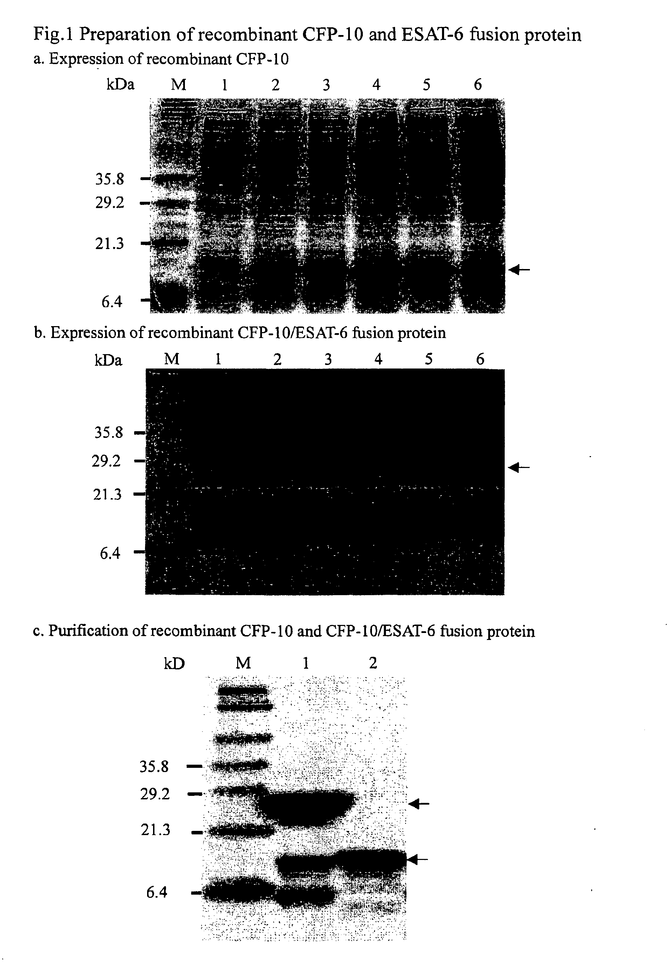

Fig. 1 shows the preparation of recombinant CFP-10 and ESAT-6 fusion protein;

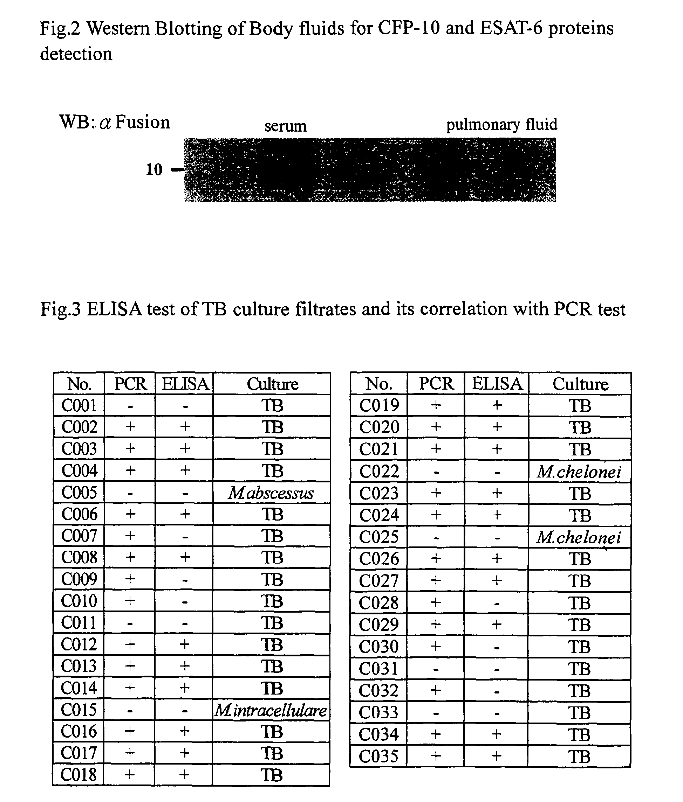

Fig. 2 shows the western blotting of body fluids for CFP-10 and ESAT-6 proteins detection;
Fig. 3 shows the ELISA test of TB culture filtrates and its correlation with PCR test;

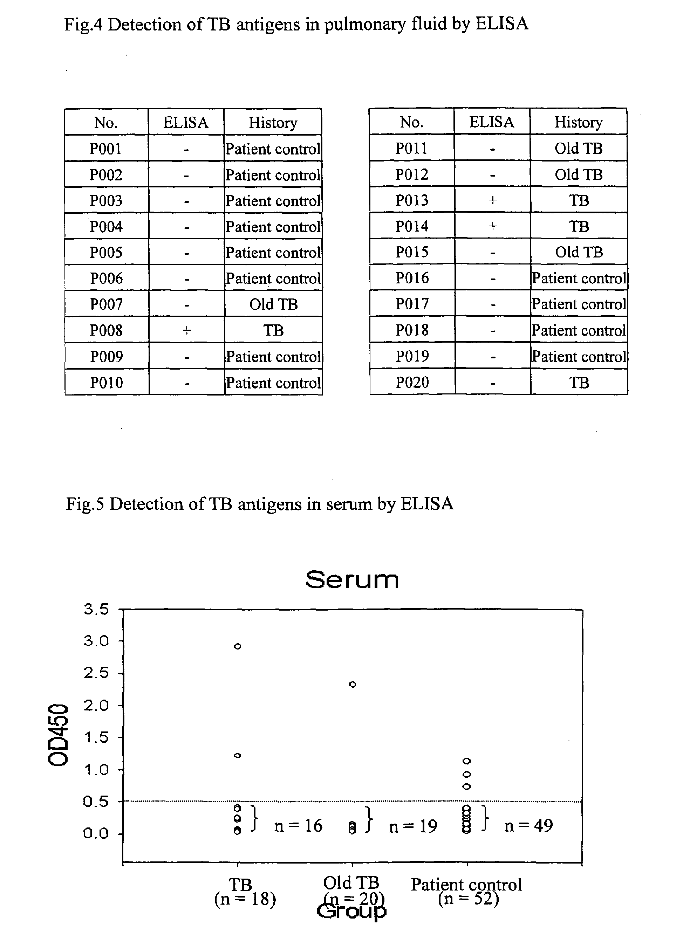

Fig. 4 shows the detection of TB antigens in pulmonary fluids by ELISA;
Fig. 5 shows the detection of TB antigens in serum by ELISA;

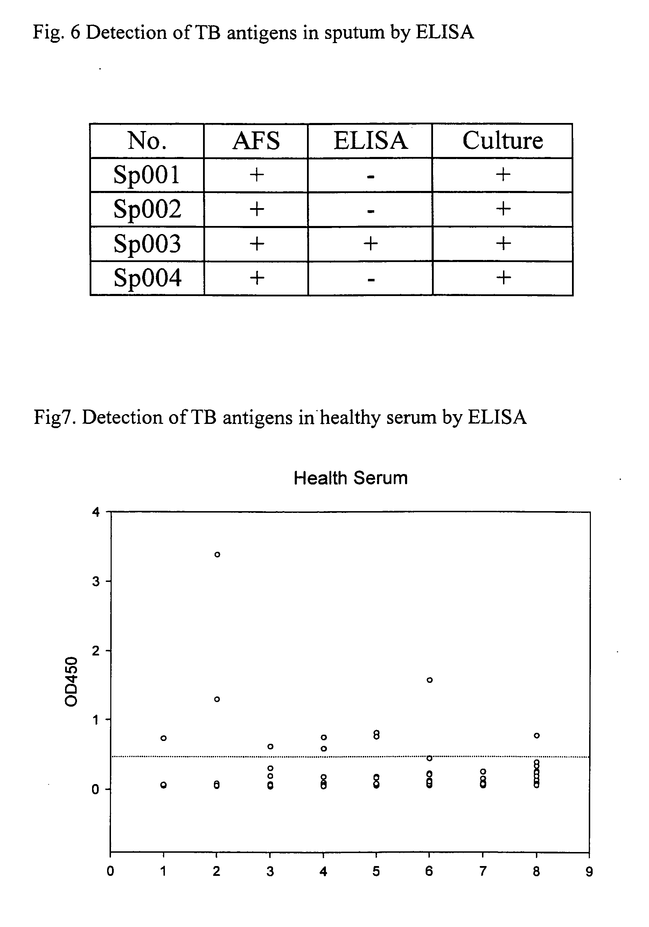

Fig. 6 shows the detection of TB antigens in sputum by ELISA; and
Fig. 7 shows the detection of TB antigens in healthy serum by ELISA.

### Preparation of the M. tuberculosis antigens

Antigens used for the immunoassays are prepared from recombinant proteins of ESAT-6, CFP-10 and CF-ES fusion proteins. Specific primers targeted to the *lhp ,esat-6* genes of M. *tuberculosis* are amplified by PCR technique. Amplified PCR products are than inserted into *p*GEM-T easy vector and transformed into *E. coli* (DH5α). Single colony of CFP-10 and ESAT-6 producing *E. coli* were selected from medium containing antibiotics. After sequencing to ensure the proper CFP-10 and ESAT-6 proteins were secreted, both CFP-10 and ESAT-6 proteins were extracted by restriction enzymes and fused together. Fused protein was reinserted into expressing vector of pET-29 system and transform into *E. coli* (BL21 DE3).

### Characterization of antigens

Most of the studies identified antigens that are shared between M. *tuberculosis,* BCG and environmental mycobacteria. Although, potentially useful in vaccine design, these antigens may not be useful in specific diagnosis of tuberculosis. Therefore, several research groups are now focusing the work to identify antigens specific for M. *tuberculosis.* Moreover, it is difficult to predict how the cross-reactive antigens will fare as vaccines in humans because among the hypotheses to explain the failure of BCG vaccines against tuberculosis is the exposure of the vaccinated population to environmental mycobacteria. In addition, the presence of cross-reactive antigens reduces the utility of PPD in the diagnosis of tuberculosis in BCG vaccinated healthy individuals. It is therefore considered that the antigens specific for M. *tuberculosis* and absent in BCG and environmental mycobacteria may provide reagents for specific diagnosis and vaccines with consistent protective efficacy.

By using techniques of biotechnology, Mahairas et al. opened a new field of study to identify *M. tuberculosis* specific antigens. They employed subtractive genomic hybridization analysis to identify genetic differences between virulent laboratory and clinical tubercle bacilli tested and was deleted only from substrains derived from the original BCG Pasteur strain after 1925. Thus the antigens encoded by the RD2 region may be useful as vaccine candidates and diagnostic reagents in areas where BCG strains lacking RD2 are used to vaccinate people against TB. MPT64, a 23KD secreted protein encoded by a gene in the RD2 region, elicits T-cell responses and cutaneous delayed type hypersensitivity (DTH) reactions in M. *tuberculosis* and their tuberculin-positive contacts respond to the antigen MPT64, but recipients of BCG vaccine strains lacking the mpt64 gene do not. Moreover, animals sensitized with BCG strains lacking the mpb64 gene (the homologue of mpt64 gene in M. *bovis)* don't respond to MPT64. The mpt64 gene is present in M. *tuberculosis* substrains H37Rv, H37Ra, and the Erdman and in the *M*. *bovis* BCG substrains Tokyo, Moreau, and Russian, whereas the *M. bovis* BCG substrains Glaxo, Pasteur, Canadian, Tice, and Danish 1331 and *M. leprae* lack the mpt64 gene. This is consistent with derived from the original BCG Pasteur strains after 1925. MPT64 has been characterized as a powerful skin test antigen which can distinguish guinea pigs immunized with *M*. *tuberculosis* from guinea pigs immunized with other mycobacteria, i.e. BCG Danish 1331 and *Mycobacterium avium*.

Among all the *M. tuberculosis* specific RD1 region gene products, the ESAT-6 (encoded by ORF7) is the most well characterized protein. The gene for ESAT-6, an early secreted antigen of *M*. *tuberculosis,* is deleted from all the BCG substrains. Native and combinant ESAT-6 are immunologically active to elicit a high level of IFN-γ from memory-immune mice challenged with *M. tuberculosis.* Analyses of subcellular fractions of *M. tuberculosis* showed the presence of ESAT-6 in cytosolicand cell wall-containing fractions as well. Several studies have shown that ESAT-6 is a major antigen of *M*. *tuberculosis* inducing protective Thl-type immune response in animals as well as in humans. Cellular immune responses to ESAT-6 discriminate between patients with pulmonary disease due to *M. tuberculosis* and *M. avium*, and thus this antigen may have potential in specific diagnosis of TB.

CFP10, a 10 kDa protein present in ST-CF of *M*. *tuberculosis* and encoded by ORF6 of RD1 region, was first identified by Berthet et al. who studied the promoter region of the esat-6 gene and found another cotranscribed gene 1 hp coding for a low molecular mass protein. Similarly, Skjot et al. have described another low-mass *M. tuberculosis* protein, TB10.4, belonging to the ESAT-6 family (EAST-6, CFP10, and TB10.4) share striking immunodominance in the human immune response against *M*. *tuberculosis* and induce high levels of INF-y. Members of this family have shown promising results to improve the specific diagnosis of TB by using serological as well as cellular methods for pulmonary and extrapulmonary disease. Furthermore, overlapping peptide pools covering the entire sequence of ESAT-6 and CFP10 are as effective in T cell responses as the respective full-length proteins, and thus the mixtures of synthetic peptides could replace the need of full-length proteins in the specific diagnosis of TB.

### Production of antibodies

### Generation and characterization of polyclonal antibodies against M. tuberculosis antigens

Antisera will be obtained from female NZW rabbits that have been immunized with *M*. *tuberculosis* antigens. The primary dose of antigens will be infected subcutaneously with complete Freund's adjuvant. Booster in incomplete Freund's adjuvant will be given at 2-week intervals. Antiserum is obtained 2 weeks after the last booster. Antibodies will be precipitated with ammonium sulfate and extensively dialyzed against PBS and stored at -20° C.

Double immuno-diffusion gels will be used in preliminary tests of the reactivity of antisera. The antisera raised against *M*. *tuberculosis* antigens from each donor will be tested against native and antigens from various donors. Quantitative estimates of antibody reactivity and specificity will be obtained by ELISA test.

### Generation and characterization of monoclonal antibodies against M. tuberculosis antigens

Monoclonal antibodies directed against specific epitopes of specific antigens will be cloned from hybridomas generated from mice that have not been immunized exogenously, as described in detail previously. By lymphocytes from the spleens of two mice will be fused with a myeloma cell line. Hybridoma supernatants will be be screened for binding to specific epitopes. Selected hybridomas will be cloned by limiting dilution, and cells will be infected intraperitonearlly into Pristane primed BALB/C mice to produce ascites fluid. After cloning, all MABs will be characterized, and purified from ascites by fast protein liquid chromatography.

A: Immunoassays□Immunochromatographic assay is a qualitative test for users without experience, single-step operation, a small sample volume required, no instrumentation required, rapid, clear, and easy-to read test result. When the liquid sample is applied to a nitrocellulose strip, the sample analyte interacts with specific color particle-conjugated antibody against *M. tuberculosis* antigens. The complex of antibody conjugate and analyte by capillary diffusion through the strip, captured at detection line where immobilized secondary antibody to another epitope of the *M. tuberculosis* antigens, producing a distinct signal in the form of a sharp color line. A control line may also be formed on the membrane by excess particle conjugate, indicating the test is validated.

B: Latex agglutination assay is a qualitative test for users without experience, single-step operation, a small sample volume required, no instrumentation required, rapid, clear, and easy-to read test result. When *M*. *tuberculosis* antigens of the body fluid encounters with the latex microparticles sensitized with *M. tuberculosis* specific antibody, an antigen-antibody reaction occurs, that results in the agglutination among the latex microparticles. The agglutination will increase the turbidity of the sample and the degree of agglutination is detected by the absorbance change. The value of the absorbance change is proportional to the antigens concentration of the sample.

C: ELISA- The immunoassays require two primary antibodies against *M. tuberculosis* antigens which must be purified by an affinity column. At least one of the primary antibodies is attached to a solid carrier. Optionally, a secondary antibody against an animal species for producing one of the primary antibodies (i.e., the unbounded primary antibody) can be added. Either the other primary antibody (i.e., the unbounded primary antibody) or the secondary antibody is labeled with a detection agent, which can be an enzymatic marker, a fluorescent or luminescent agent, a radioactive label, or a color particle. The body fluids may be used directly (serum, SCF, urine, sputum), concentrated (lung fluids, urine), diluted (serum, stool).

D: Immuno-chips, Specific *M. tuberculosis* antigens can be immobilized on the electric devices (transducers), such as piezoelectric crystal, surface plasmon resonance transducer, surface acoustic resonance transducer, electrodes, caloric meter, and other light detecting devices. When *M. tuberculosis* antigens of the body fluid encounters with the immobilized *M. tuberculosis* specific antibody, an antigen-antibody reaction occurs, that results in change of electric signal.

## Claims

1. A method for detection of mycobacterium tuberculosis antigens in biological fluids comprising:
(a) polyclonal and monoclonal antibodies against the specific proteins of *Mycobacterium tuberculosis;* and
(b) at least one detection device capable of being used to determine the amounts of antibodies-bound *Mycobacterium tuberculosis* antigens.

2. A device for detection of mycobacterium tuberculosis antigens in biological fluids comprising:
(a) polyclonal and monoclonal antibodies against the specific proteins of *Mycobacterium tuberculosis;* and
(b) at least one detection device capable of being used to determine the amounts of antibodies-bound *Mycobacterium tuberculosis* antigens.

3. The device as claimed in claim 2, wherein the specific proteins of *Mycobacterium tuberculosis* include the proteins encoded by RD1, RD2, and RD3 gene clusters of *Mycobacterium tuberculosis,* synthetic peptides of RDs regions, and any antigenic fragments of those proteins by preparing both from culture purification and genetic engineering processes.

4. The device as claimed in claim 2 or 3, wherein the preferred specific proteins of *M. tuberculosi* are culture filtrated protein-10 (CFP10), early secretion antigen-6 (ESAT-6), and CFP10/ESAT-6 fusion proteins encoded from the RD1 of *M. tuberculosis.*

5. The device as claimed in claim 2, wherein the preferred antibodies are derived from CFP-10 and ESAT-6, and CFP10/ESAT-6 fusion proteins.

6. The device as claimed in one of claims 2 to 5, wherein the detection devices include radio-immunoassay, enzyme immunoassay, immunochromatographic assay device, fluorescence immunoassays, immunoblotting and dot immunobinging, luminescence immunoassay, latex-agglutination, antibody chips.

7. The device as claimed in claim 2 being capable of being used to detect the *Mycobacterium tuberculosis* antigens in blood, blood plasma, blood serum, sputum, urine, cerebrospinal fluid, pleural effusion, culture filtrates, and tissues fluids.

8. The device as claimed in claim 2 comprising at least one reagent selected from the group consisting of:
(a) an immobilized antibody capable of capturing synthetic peptides and antigens encoded by RD1, RD2, and RD3 gene clusters of *Mycobacterium tuberculosis;*
(b) the secondary antibody being labeled with a detection agent, which can be an enzymatic marker, a fluorescent or luminescent agent, a radioactive label, or a color particle.
(c) Recombinant antigen as a calibration standard and positive control.

9. The device as claimed in claim 2 further comprising at least one reagent selected from the group consisting of:
(a) an immobilized antibody being capable of capturing synthetic peptides and antigens encoded by RD1, RD2, and RD3 gene clusters of *Mycobacterium tuberculosis;*
(b) the materials for the antibody immobilization including particles such as micro-titer plates, color latex beads, gold colloidal beads, and magnetic beads; or biosensor transducer such as piezoelectric sensor, surface wave acoustic sensor, electrodes, semiconductor, light detecting devices; and
(c) recombinant antigen as a calibration standard and positive control.
